(19) Europäisches Patentamt
European Patent Office
Office européen des brevets

(11) EP 2 960 237 A1

(12) **EUROPEAN PATENT APPLICATION**

(43) Date of publication:
**30.12.2015 Bulletin 2015/53**

(21) Application number: **15174323.4**

(22) Date of filing: **13.12.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2011 JP 2011275819**

(62) Document number(s) of the earlier application(s) in accordance with Art. 76 EPC:
**12858308.5 / 2 792 676**

(51) Int Cl.:
***C07D 309/28*** *(2006.01)*

(71) Applicant: **Daiichi Sankyo Company, Limited**
**Toyko 103-8426 (JP)**

(72) Inventors:
- **TORIYAMA, Fumihiko**
**Hiratsuka-shi, Kanagawa 254-0014 (JP)**
- **KUWAHARA, Yasuhisa**
**Odawara-shi, Kanagawa 250-0216 (JP)**

(74) Representative: **Fairbairn, Angus Chisholm**
**Marks & Clerk LLP**
**90 Long Acre**
**London**
**WC2E 9RA (GB)**

Remarks:
This application was filed on 29-06-2015 as a divisional application to the application mentioned under INID code 62.

(54) **METHOD FOR PRODUCING NEURAMINIC ACID DERIVATIVE**

(57) The present invention provides a method for manufacturing a compound (10):

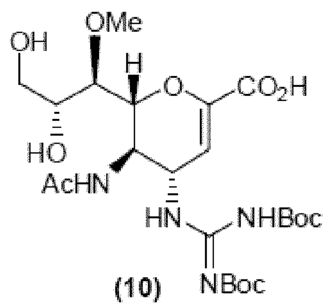

**(10)**

by reacting a compound (7):

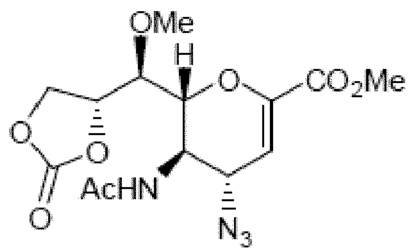

**(7)**



Printed by Jouve, 75001 PARIS (FR)

(Cont. next page)

with triphenylphosphine followed by reacting with a base and water to produce a compound (8):

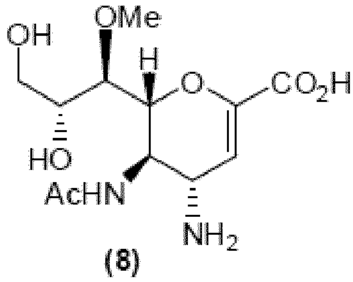

(8)

,

and adding an acid to an aqueous solution containing the compound (8) to remove carbonic acid and reacting the compound represented by the formula (8) with a compound (9):

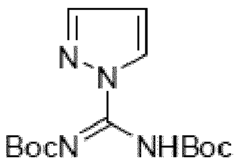

(9)

in the aqueous solution from which carbonic acid has been removed.

## Description

TECHNICAL FIELD

The present invention relates to a method for manufacturing neuraminic acid derivatives having neuraminidase inhibitory activity.

BACKGROUND ART

A compound represented by the following formula:

[Chemical Formula 1]

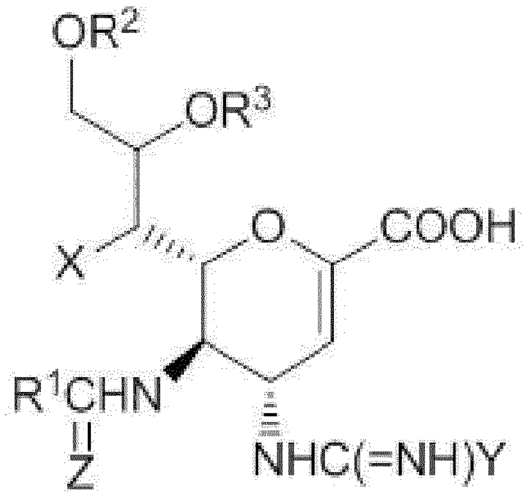

[wherein $R^1$ represents an alkyl group having 1 to 4 carbon atoms, and the like, $R^2$ and $R^3$ may be the same or different and represent a hydrogen atom or an aliphatic acyl group having 2 to 25 carbon atoms, X represents a hydroxyl group, an alkoxy group having 1 to 4 carbon atoms, and the like, Y represents $NH_2$, and the like and Z represents an oxygen atom, and the like], or a pharmacologically acceptable salt thereof, is known to have superior neuraminidase inhibitory activity and be useful as a drug for treatment or prevention of influenza (Patent Documents 1, 2 and 3).

Process A is known as a method for manufacturing a compound represented by the formula (I) that is embraced within the compound represented by the aforementioned formula or a pharmacologically acceptable salt thereof (provided that the compound represented by the formula (I) may contain a regioisomer in the form of a compound represented by the formula (II)) :

[Chemical Formula 2]

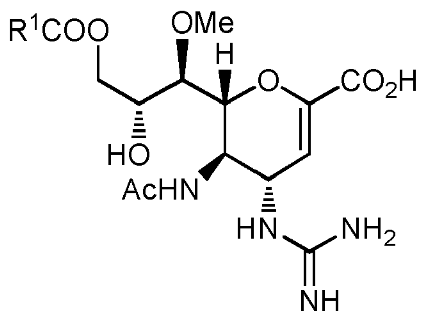

(I)

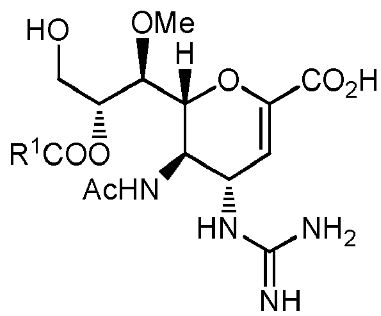

(II)

[wherein $R^1$ represents an alkyl group having 1 to 19 carbon atoms]) (Patent Document 4).

[Chemical Formula 3]

Process A

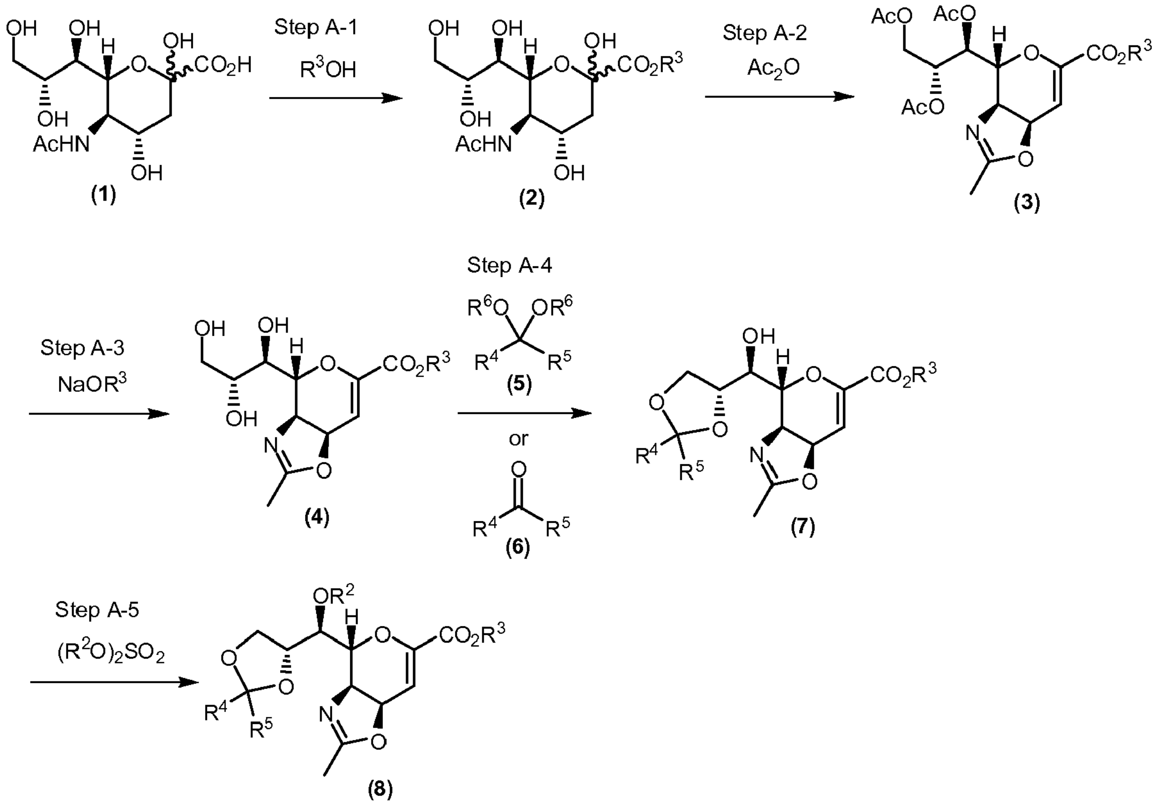


[Chemical Formula 4]

Process A (continued)

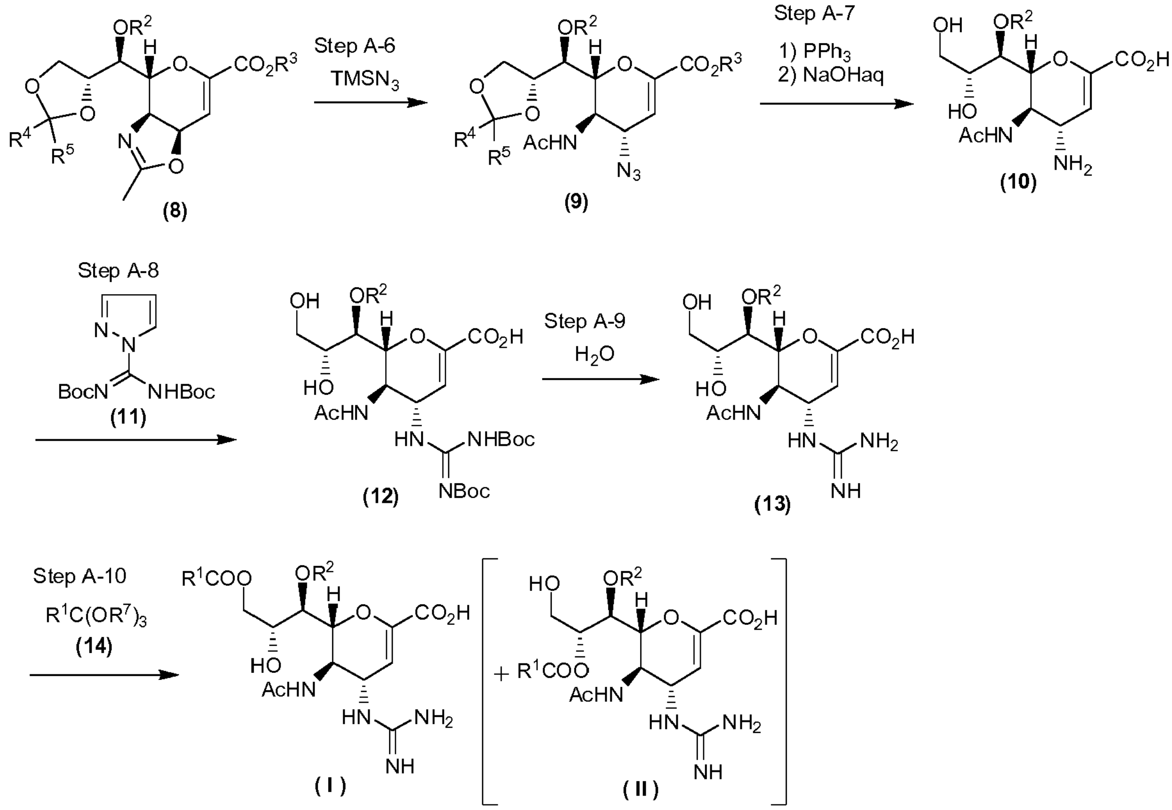


(8) (9) (10) (11) (12) (13) (14) (I) (II)

In the scheme of the aforementioned Process A, $R^1$ represents a $C_1$-$C_{19}$ alkyl group, $R^2$ represents a $C_1$-$C_4$ alkyl group, $R^3$, $R^6$ and $R^7$ independently represent a $C_1$-$C_6$ alkyl group, $R^4$ and $R^5$ independently represent a hydrogen atom, a $C_1$-$C_6$ alkyl group or a phenyl group, or $R^4$ and $R^5$ together form a tetramethylene group, a pentamethylene group or an oxo group.

Namely, in Process A, known compound (1) is reacted with an alcohol having the formula $R^3OH$ in the presence of an acid to produce compound (2) (Step A-1), compound (2) is reacted with acetic anhydride in the presence of an acid to produce compound (3) (Step A-2), compound (3) is reacted with a compound having the formula $NaOR^3$ to produce compound (4) (Step A-3), compound (4) is reacted with compound (5) or compound (6) to produce compound (7) (Step A-4), compound (7) is reacted with a compound having the formula $(R^3O)_2SO_2$ in the presence of a base to produce compound (8) (Step A-5), compound (8) is reacted with azidotrimethylsilane in the presence of a Lewis acid to produce compound (9) (Step A-6), compound (9) is treated with triphenylphosphine (Step A-7a), the compound obtained in Step A-7a is treated with a base and water (Step A-7b) to produce compound (10) (Step A-7), compound (10) is reacted with compound (11) to produce compound (12) (Step A-8), compound (12) is reacted with water to produce compound (13) (Step A-9), and compound (13) is reacted with compound (14) in the presence of an acid to produce compound (I) (Step A-10) [which may include the compound represented by the formula (II)].

In the aforementioned scheme of Process A, a trifluoroacetic acid salt of a compound represented by the formula (13):

[Chemical Formula 5]

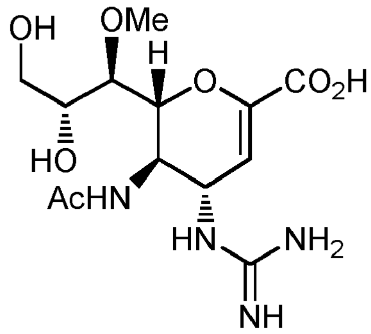

is also known to have superior neuraminidase inhibitory activity and be useful as a drug for treatment or prevention of influenza (Non-Patent Document 1 or 2).

In the aforementioned Process A of Patent Document 4, compound (9) is produced by reacting compound (8) with azidotrimethylsilane in the presence of a Lewis acid in Step A-6. Since it is necessary to decompose residual azidotrimethylsilane, a compound for decomposing the residual azidotrimethylsilane is added in the form of an aqueous solution. However, in the case of using titanium (IV) isopropoxide as a preferred example of a Lewis acid (see paragraph [0206] and Example 1 (Step A-6) in the specification of Patent Document 4), insoluble matter which is hardly soluble, derived from the titanium (IV) isopropoxide is formed when water is present, thereby impairing separation from compound (9). In the past, titanium tetraalkoxide was known to exist in a stable state in an aqueous solution at room temperature when converted to a chelate compound synthesized with a hydroxycarboxylic acid (Non-Patent Document 3). When using the titanium compound to denature a polymer compound having a functional group capable of reacting with the titanium compound, such as a hydroxyl group, a carboxyl group or an ester group thereof, an acetate group or an epoxy group, this type of chelate compound was used to stabilize the resulting polymer composition.

In addition, in the aforementioned Process A of Patent Document 4, there was also the problem of a lack of consistency in the production time since the reaction rate during the reaction of compound (10) with compound (11) in Step A-8 was not constant.

Prior Art Documents

Patent Documents

Patent Document 1: US Patent No. 6340702 specification (corresponding to Japanese Patent No. 3209946)
Patent Document 2: US Patent No. 6844363 specification (corresponding to Japanese Patent No. 3920041)
Patent Document 3: International Publication No. WO 01/80892 pamphlet (corresponding to Japanese Patent No. 4205314)
Patent Document 4: US Patent Application Publication No. 12/450,699 specification (corresponding to International Publication No. 2008/126943 pamphlet)

Non-Patent Documents

Non-Patent Document 1: T. Honda, et al., Bioorganic Medicinal Chemistry Letters, 2002, pp. 1921-1924
Non-Patent Document 2: T. Honda, et al., Bioorganic Medicinal Chemistry Letters, 2002, pp. 1925-1928
Non-Patent Document 3: Japanese Patent Application (Kokai) No. Sho 49-94768

SUMMARY OF THE INVENTION

Problems to be Solved by the Invention

As a result of conducting extensive studies on a method for manufacturing neuraminic acid derivatives having neuraminidase inhibitory activity, the inventors of the present invention found that the manufacturing method of the

present invention is superior to known manufacturing methods from an industrial viewpoint, thereby leading to completion of the present invention.

Means for Solving the Problems

The present invention provides a method for manufacturing neuraminic acid derivatives.
Namely, the present invention is:

[1] a method for manufacturing compounds represented by the formula (I), or a pharmacologically acceptable salt thereof, as indicated by the following production scheme:

[Chemical Formula 6]

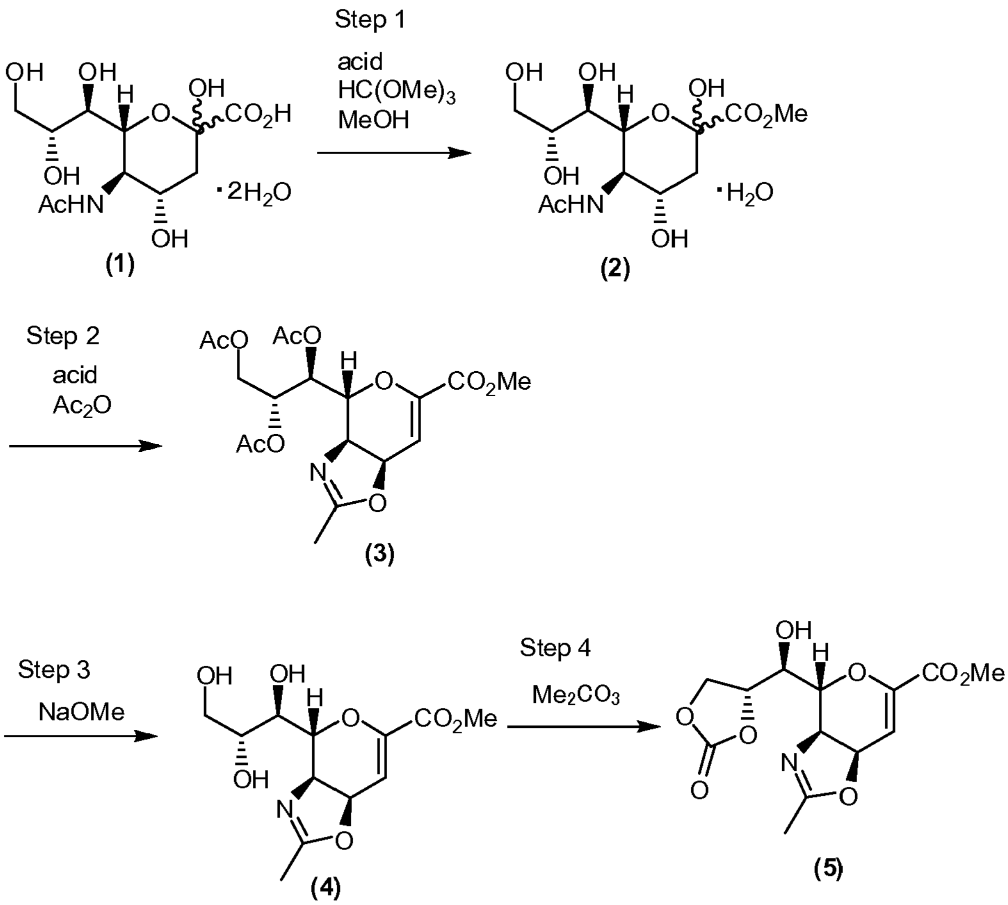


(1) (2) (3) (4) (5)

[Chemical Formula 7]

(continued)

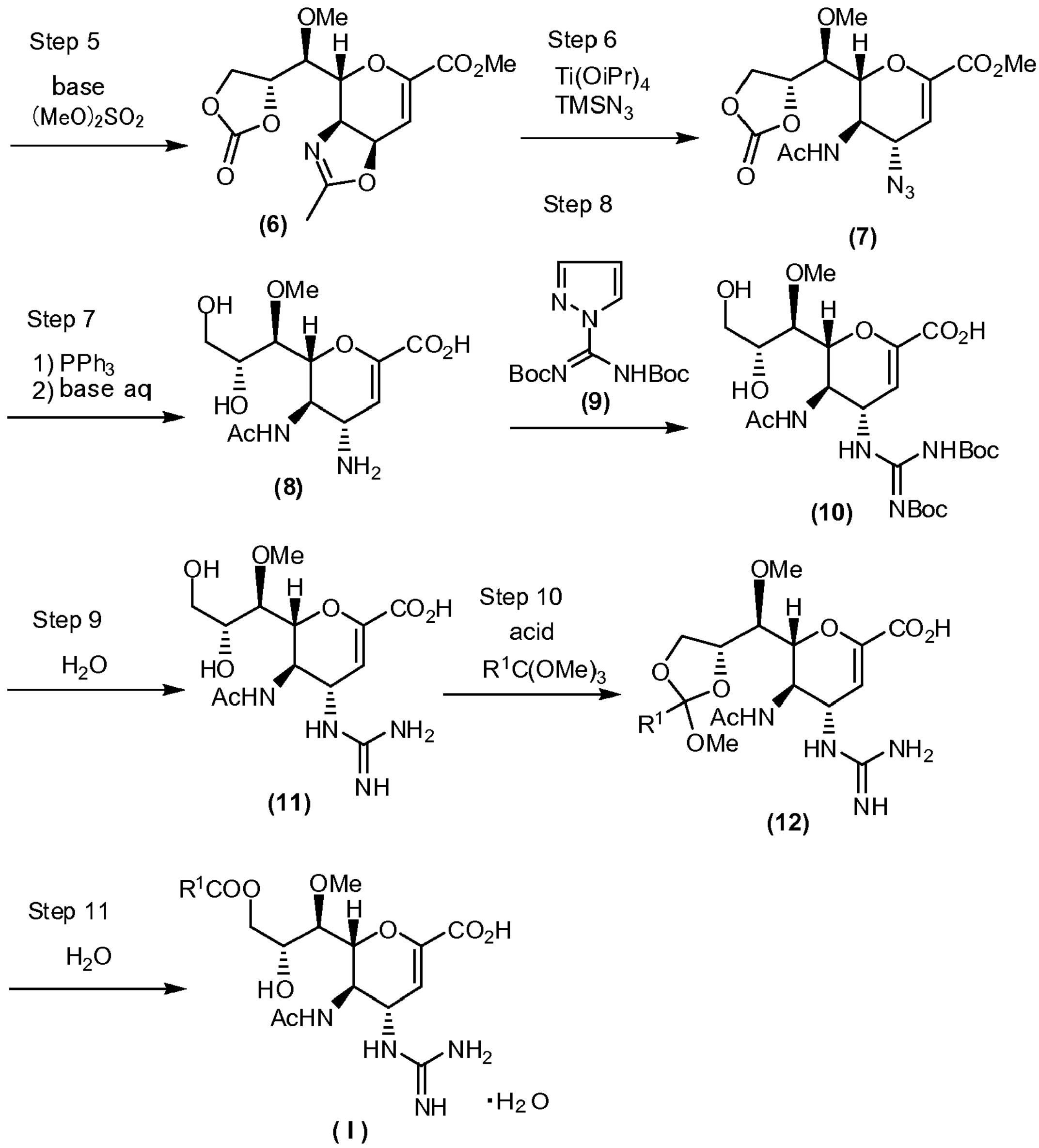


(6) (7) (8) (9) (10) (11) (12) (I)

[wherein $R^1$ represents a $C_1$-$C_{19}$ alkyl group], comprising:

reacting a compound represented by the formula (1) with methanol in the presence of an acid and a compound represented by the formula $HC(OMe)_3$ to produce a compound represented by the formula (2),
then reacting the compound represented by the formula (2) with acetic anhydride in the presence of an acid to produce a compound represented by the formula (3),
then reacting the compound represented by the formula (3) with sodium methoxide to produce a compound represented by the formula (4),
then reacting the compound represented by the formula (4) with dimethyl carbonate to produce a compound represented by the formula (5),
then reacting the compound represented by the formula (5) with dimethyl sulfate in the presence of a base to produce a compound represented by the formula (6),
then reacting the compound represented by the formula (6) with azidotrimethylsilane in the presence of titanium (IV) isopropoxide to produce a compound represented by the formula (7),
then reacting the compound represented by the formula (7) with triphenylphosphine followed by reacting with a base and water to produce a compound represented by the formula (8),
then reacting the compound represented by the formula (8) with a compound represented by the formula (9) to

produce a compound represented by the formula (10),

then heating the compound represented by the formula (10) in water to produce a compound represented by the formula (11),

then reacting the compound represented by the formula (11) with a compound represented by the formula $R^1C(OMe)_3$ [wherein $R^1$ represents a $C_1$-$C_{19}$ alkyl group] in the presence of an acid to produce a compound represented by the formula (12), and

then reacting the compound represented by the formula (12) with water to produce a compound represented by the formula (I) or a pharmacologically acceptable salt thereof.

The present invention further provides the manufacturing methods indicated below.

[2] A method for manufacturing a compound represented by the formula (2) by reacting a compound represented by the formula (1) with methanol in the presence of an acid and a compound represented by the formula $HC(OMe)_3$.

[3] A method for manufacturing a compound represented by the formula (I), or a pharmacologically acceptable salt thereof, characterized by comprising the manufacturing method described in [2] above.

[4] A method for manufacturing a compound represented by the formula (7), wherein a compound represented by the formula (6) is reacted with azidotrimethylsilane in the presence of titanium (IV) isopropoxide to crystallize a compound represented by the formula (7) in the resulting reaction solution, followed by adding hydroxycarboxylic acid and then an aqueous sodium nitrite solution to the reaction solution and isolating the compound represented by the formula (7) from the reaction solution.

[5] A method for manufacturing a compound represented by the formula (I), or a pharmacologically acceptable salt thereof, characterized by comprising the manufacturing method described in [4] above.

[6] A method for manufacturing a compound represented by the formula (10) by reacting a compound represented by the formula (7) with triphenylphosphine followed by reacting with a base and water to produce a compound represented by the formula (8), and adding an acid to an aqueous solution containing the compound represented by the formula (8) to remove carbonic acid and reacting the compound represented by the formula (8) with a compound represented by the formula (9) in the aqueous solution from which carbonic acid has been removed.

[7] A method for manufacturing a compound represented by the formula (I), or a pharmacologically acceptable salt thereof, characterized by comprising the manufacturing method described in [6] above.

[8] A manufacturing method of any one of [1], [3], [5] and [7] above, wherein $R^1$ is a 1-heptyl group.

[9] A compound represented by the formula (I), or a pharmacologically acceptable salt thereof, obtained according to the manufacturing method described in any one of [1], [3], [5], [7] and [8] above, wherein the 50% by weight particle diameter as determined by laser diffraction/scattering particle size distribution measurement is 5 $\mu$M to 15 $\mu$M, and the 90% by weight particle diameter as determined by laser diffraction/ scattering particle size distribution measurement is 15 $\mu$M to 35 $\mu$M.

In the present invention, "$C_1$-$C_{19}$ alkyl group" of $R^1$ represents a linear or branched alkyl group having 1 to 19 carbon atoms, and may be, for example, a methyl group, ethyl group, propyl group, butyl group, pentyl group, hexyl group, heptyl group, octyl group, nonyl group, decanyl group, undecanyl group, dodecanyl group, tridecanyl group, tetradecanyl group, pentadecanyl group, hexadecanyl group, heptadecanyl group, octadecanyl group or nonadecanyl group, preferably a $C_5$-$C_{19}$ alkyl group, more preferably a $C_5$-$C_{17}$ alkyl group, even more preferably a pentyl group, heptyl group, nonyl group, undecanyl group, tridecanyl group, pentadecanyl group or heptadecanyl group, further preferably a 1-pentyl group, 1-heptyl group, 1-nonyl group, 1-undecanyl group, 1-tridecanyl group, 1-pentadecanyl group or 1-heptadecanyl group, and most preferably a 1-heptyl group.

In the present invention, "pharmacologically acceptable salt" may be, for example, a hydrohalic acid salt such as hydrofluoric acid salt, hydrochloric acid salt, hydrobromic acid salt and hydroiodic acid salt; an inorganic acid salt such as nitric acid salt, perchloric acid salt, sulfuric acid salt and phosphoric acid salt; an alkanesulfonic acid salt such as methanesulfonic acid salt, ethanesulfonic acid salt and trifluoromethanesulfonic acid salt; an arylsulfonic acid salt such as benzenesulfonic acid salt and p-toluenesulfonic acid salt; an organic acid salt such as acetic acid salt, trifluoroacetic acid salt, citric acid salt, tartaric acid salt, oxalic acid salt and maleic acid salt; an amino acid salt such as glycine salt, lysine salt, arginine salt, ornithine salt, glutamic acid salt and aspartic acid salt; an alkali metal salt such as lithium salt, sodium salt and potassium salt; an alkaline earth metal salt such as calcium salt and magnesium salt; a metal salt such as aluminum salt, iron salt, zinc salt, copper salt, nickel salt and cobalt salt; or an organic amine salt or organic ammonium salt such as ammonium salt, t-octylamine salt, dibenzylamine salt, morpholine salt, glucosamine salt, ethylenediamine salt, guanidine salt, diethylamine salt, triethylamine salt, dicyclohexylamine salt, procaine salt, ethanolamine salt, diethanolamine salt, piperazine salt and tetramethyl ammonium salt.

Compound (I) produced according to the manufacturing method of the present invention can be present together with the aforementioned compound (II) in the form of a regioisomer having a different acyloxy group substitution site.

The compounds related to the present invention have asymmetric carbons within their molecule, and thus there

exist stereoisomers (enantiomers and diastereomers are included). These stereoisomers and mixtures thereof in arbitrary ratios (including racemic form) are embraced in the compounds of the present invention.

It is known that when compound (I) is administered to a warm-blooded animal, the acyloxy group at the 3-position of the side chain is converted into a hydroxyl group by a metabolic reaction such as hydrolysis, and the generated compound (11):

[Chemical Formula 8]

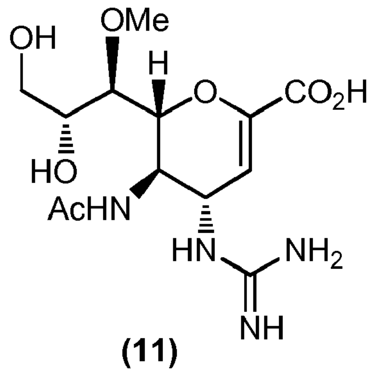

**(11)**

shows pharmacological activity (Patent Document 1 and the like). In addition, when compound (II) is administered to a warm-blooded animal, the acyloxy group at the 2-position of the side chain is converted into a hydroxyl group by a metabolic reaction such as hydrolysis, and compound (11) is generated in a similar manner. Since both compound (I) and compound (II) are converted into the same compound (11), which is the active metabolite, within the organism of a warm-blooded animal, it can be considered that both compounds are active ingredients, from the point of view of using a mixture of compound (I) and compound (II) as a medicament.

In the present invention, the chemical purity of the compound, the content of a compound as an impurity, the composition ratio of stereoisomers, or the composition ratio of a mixture of compound (I) and compound (II) may be determined by well-known methods in the field of organic chemistry (for example, high-performance liquid chromatography, weight percent, etc.), and is preferably determined by peak area ratios under high-performance liquid chromatography (hereinafter also referred to as HPLC). The measurement conditions for HPLC will be suitably selected; however, they are preferably as shown herein below.

HPLC Measurement Conditions (1)

Column: Column packed with octadecylsilylated silica gel 5 μm for use in liquid chromatography in a stainless steel tube having an inner diameter of 4.6 mm and length of 25 cm (for example, the L-Column ODS manufactured by the Chemicals Evaluation and Research Institute, Japan, 4.6 x 250 mm, 5 μm)
Column temperature: 30°C
Measurement wavelength: 233 nm
Mobile phase: Constant at mobile phase A:mobile phase B = 70:30
Mobile phase A: 0.01 mol/L phosphate buffer solution (pH 3)
Mobile phase B: Acetonitrile
(Note, however, that, 0.01 mol/L phosphate buffer solution (pH 3) of mobile phase A is a buffer solution prepared by adding 0.01 mol/L phosphoric acid to a 0.01 mol/L aqueous potassium dihydrogenphosphate solution and adjusting the pH to 3.)
Flow rate: approximately 1 mL/min
Sample concentration: approximately 100 μg/L
Injection amount: 10 μL
Peak detection range: From 0 minutes to roughly 2.3 times the length of retention time of compound (I)

HPLC Measurement Conditions (2)

Column: Column packed with octadecylsilylated silica gel 3μ m for use in liquid chromatography in a stainless steel tube having an inner diameter of 4.6 mm and length of 15 cm (for example, Hydrosphere C-18 manufactured by YMC, 4.6 x 150 mm, 3 μm)
Column temperature: 20°C
Measurement wavelength: 233 nm
Mobile phase A: 0.01 mol/L phosphate buffer solution (pH 3)
Mobile phase B: Acetonitrile/methanol mixture (7:3)

Gradient conditions:

0 to 5 min: mobile phase A: mobile phase B = 100:0
5 to 15 min: mobile phase ratio changed to mobile phase A: mobile phase B = 75:25
15 to 65 min: mobile phase A: mobile phase B = 75:25
65 to 75 min: mobile phase ratio changed to mobile phase A: mobile phase B = 45:55
75 to 105 min: mobile phase A: mobile phase B = 45:55
(Note, however, that, the 0.01 mol/L phosphate buffer solution (pH 3) of mobile phase A indicates a buffer solution prepared by adding 0.01 mol/L phosphoric acid to a 0.01 mol/L aqueous potassium dihydrogenphosphate solution and adjusting the pH to 3.)
Flow rate: approximately 1.1 mL/min
Sample concentration: approximately 1000 μg/L
Injection amount: 5 μL
Range detected with peak: To approximately 1.8 times the length of retention time of compound (I)

By HPLC measurement conditions (1), the peak area ratios of compound (I) and compound (II) detected from 0 minutes to approximately 2.3 times the length of retention time of compound (I) are measured. By HPLC measurement conditions (2), the peak area ratio of compounds as impurities, which are detected from 0 minutes to approximately 1.8 times the length of retention time of compound (I) is measured. Here, the peaks of the compounds as impurities indicate the peaks when the peak of compound (I), the peak of compound (II), and the peaks detected when solvent alone is injected (for example, the peak of solvent and the peak derived from noise), are subtracted from all of the peaks that are detected as 0.01% or more.
The chemical purity (%) of compound (I) can be calculated according to the following equation.

Chemical purity of compound (I)
= 100 - sum of peak area ratio (%) of compound as impurity

According to the manufacturing method of the present invention, compound (II) may also be produced in addition to compound (I), and in the case of additionally producing compound (II) together with compound (I), chemical purity is calculated as the mixture of compound (I) and compound (II).
The peak area ratios of compound (I) and compound (II) can be measured according to the aforementioned HPLC measurement conditions (1). The composition ratio of a mixture of compound (I) and compound (II) can be calculated according to the following equation.

Composition ratio of compound (I)
= [Peak area of compound (I)/[peak area of compound (I) + peak area of compound (II)]] × 100

Composition ratio of compound (II)
= [Peak area of compound (II)/[peak area of compound (I) + peak area of compound (II)]] × 100

The manufacturing method of the present invention as described above is superior to the known excellent

manufacturing method in the form of Process A described in Patent Document 4 with respect to, for example, the points indicated below. (1) The starting material is a dihydrate of compound (1).

In Process A described in Patent Document 4, an anhydride is used as the starting material in the form of compound (1). In contrast, a dihydrate is used in the manufacturing method of the present invention.

A dihydrate has a smaller specific volume than an anhydride. In contrast to the specific volume of an anhydride being 6 $cm^3/g$ to 8 $cm^3/g$, the specific volume of a dihydrate is 1 $cm^3/g$ to 2 $cm^3/g$. Consequently, when a dihydrate is used as the starting material, operability and workability during production are superior to those of an anhydride. In addition, the use of a dihydrate makes it possible to reduce the amount of solvent used in Step 1 in comparison with the case of using an anhydride.

Since the amount of solvent can be reduced, the concentration of the reaction substrate in Step 1 increases, thereby making it possible to improve the reactivity of Step 1 in comparison with the case of using an anhydride. Consequently, the reaction temperature of Step 1 can be lowered and reaction time can be shortened. In addition, since the reaction temperature can be lowered, decomposition of compound (2) formed in the reaction solution can be inhibited.

Since the amount of solvent used can be reduced, a procedure for concentrating the solvent after the reaction can be omitted.

Since the amount of solvent used can be reduced, the amount of poor solvent used when crystallizing compound (2) can also be reduced.

Since the use of a dihydrate improves solubility, the reaction proceeds more easily in comparison with the case of using an anhydride.

(2) Step 8

The reaction raw materials in the form of compound (8) and compound (9) of Step 8 of the present invention:

[Chemical Formula 9]

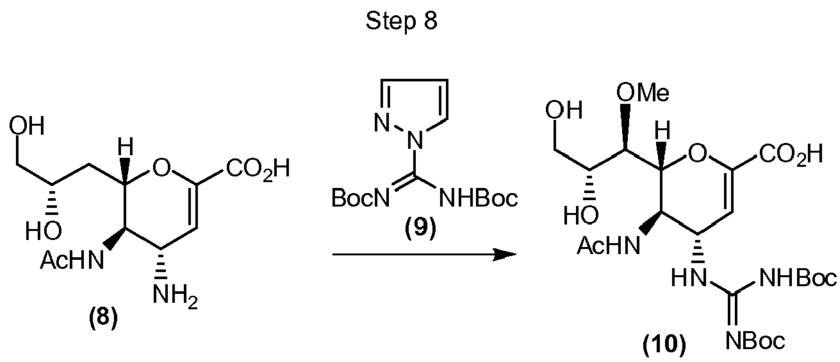


and the reaction product of Step (8) in the form of compound (10) are compounds corresponding to the reaction raw materials of compound (10) and compound (11) of Step A-8 of Process A described in Patent Document 4, and the reaction product of compound (12) in Step A-8, respectively.

Step A-8 of Example 1 of Patent Document 4 discloses a specific method for producing compound (12). According to this method:

[1] N,N'-bis(tert-butoxycarbonyl)-1H-pyrazole-1-carboximidamide is added to an aqueous solution of compound (10) obtained in Step A-7 at room temperature followed by stirring at the same temperature;

[2] water is added to the resulting reaction solution followed by adjusting the pH to 8.35 with concentrated hydrochloric acid; and,

[3] the solvent is distilled off under reduced pressure, the resulting solution is repeatedly washed with ethyl acetate to separate the aqueous layer, and the reaction product of Step A-8 in the form of compound (12) is extracted from this aqueous layer.

In contrast, in Step 8 of the present invention, carbonic acid formed as a by-product in Step 7 carried out prior to Step 8 is released in the form of carbon dioxide gas by first adding an acid to the aqueous solution containing compound (8) obtained following synthesis of compound (8) to adjust the pH of the aqueous solution to the acidic side. Although the reaction rate between compound (8) and compound (9) tends to not be constant if carbonic acid is allowed to remain in the system, by removing the carbonic acid by adjusting the pH, the reaction rate between compound (8) and compound

(9) is stabilized, thereby making it possible to achieve a constant reaction time.

Effects of the Invention

As a result of having superior operability of the starting material, a milder reaction temperature, a shorter reaction time and so on in comparison with known manufacturing methods, the manufacturing method of the present invention is superior for use as an industrial manufacturing method on an actual production scale. The manufacturing method of the present invention allows the obtaining of a highly pure neuraminic acid derivative at high yield.

<u>MODE FOR CARRYING OUT THE INVENTION</u>

In the present invention, neuraminic acid derivatives are produced according to the manufacturing method indicated below.

[Chemical Formula 10]

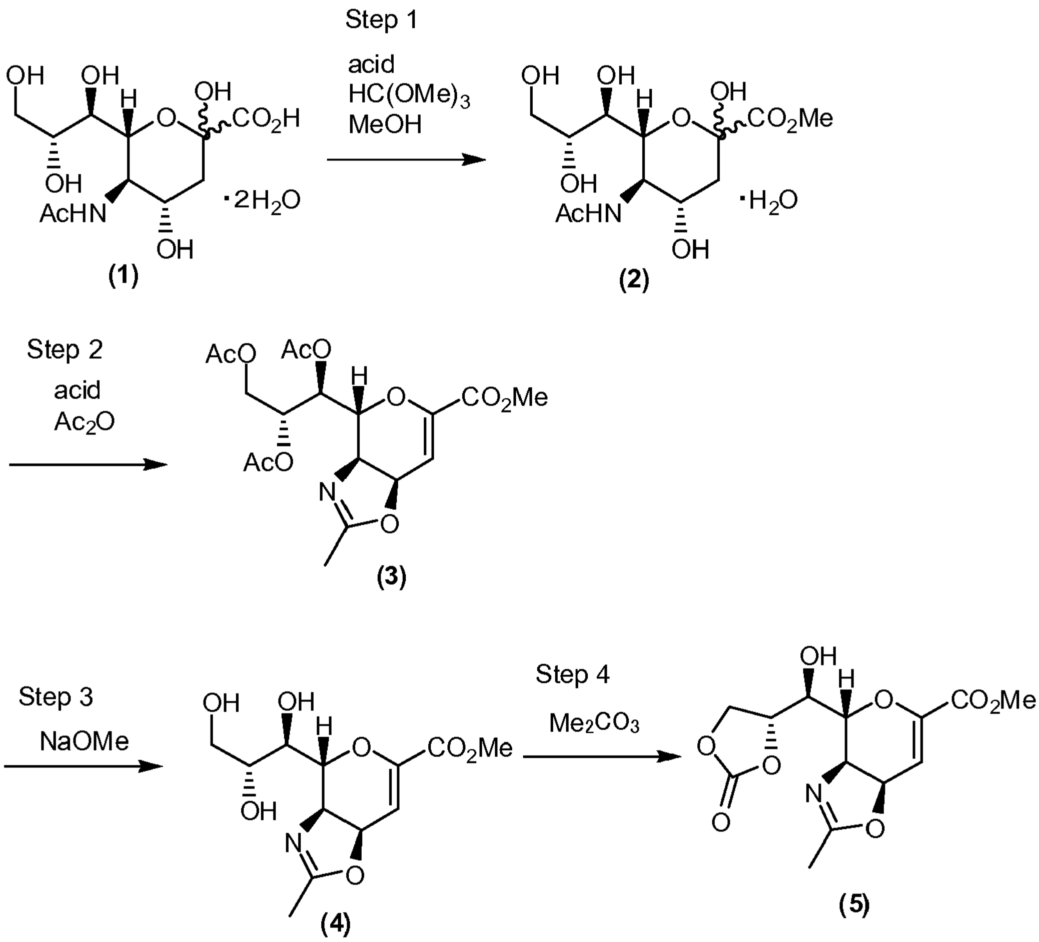


(1)

(2)

(3)

(4)

(5)

[Chemical Formula 11]

(continued)

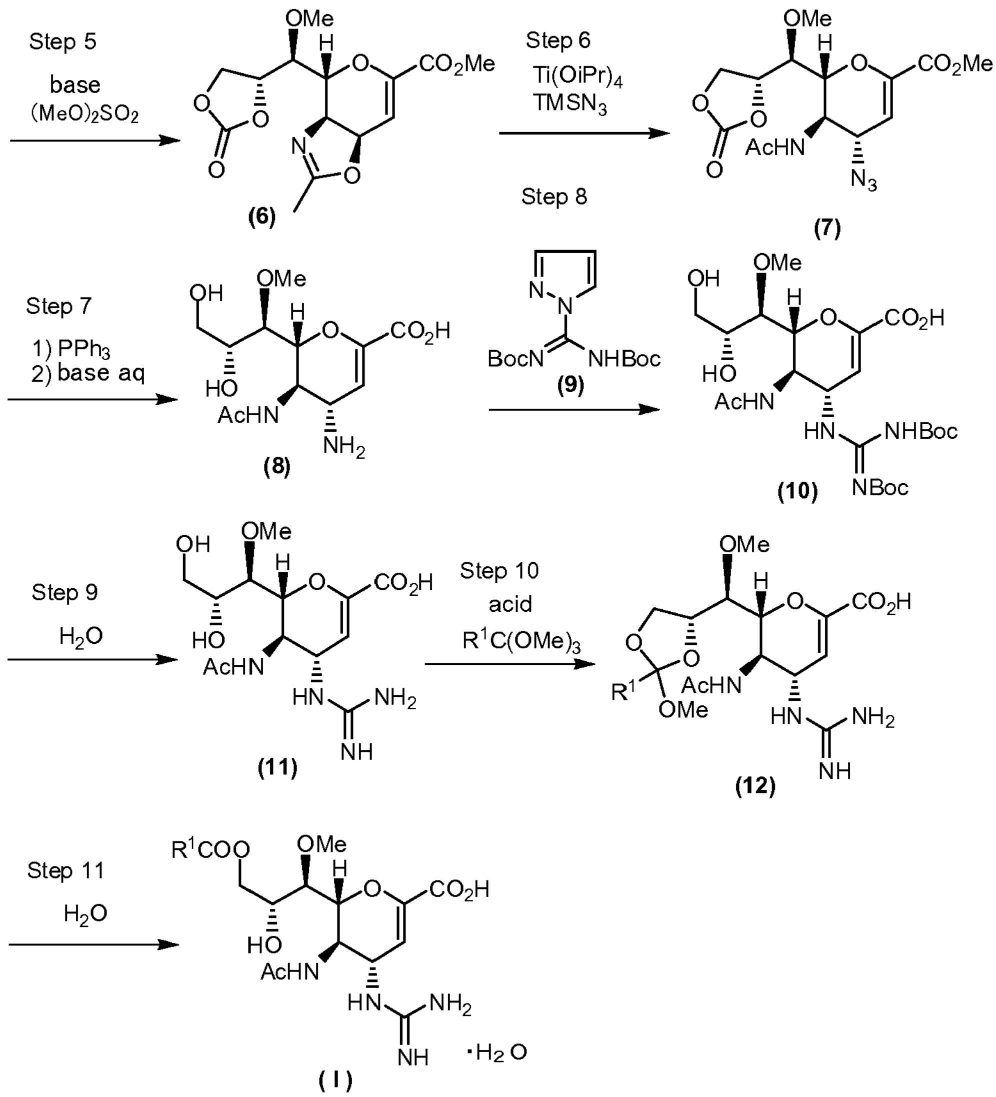


$R^1$ represents a $C_1$-$C_{19}$ alkyl group.

(Step 1)

Step 1 is a step for producing compound (2) by reacting a known compound (1) with methanol in the presence of an acid and a compound represented by the formula $HC(OMe)_3$ (trimethyl orthoformate).

There are no limitations on the order in which the acid and trimethyl orthoformate are added. They are preferably added in the order of acid and then trimethyl orthoformate.

There are no limitations on the acid used provided it can be used in an esterification reaction of a carboxyl group that uses an alcohol, and the acid can be, for example, an organic acid such as acetic acid, propionic acid, trifluoroacetic acid and pentafluoropropionic acid, an organic sulfonic acid such as p-toluenesulfonic acid, camphorsulfonic acid and trifluoromethanesulfonic acid, or an inorganic acid such as hydrogen chloride, hydrogen bromide, hydrogen iodide, phosphoric acid, sulfuric acid and nitric acid, is preferably an inorganic acid, and is most preferably sulfuric acid.

The reaction temperature is 0°C to 60°C and preferably 20°C to 40°C.

The reaction time is 30 minutes to 10 hours and preferably 1 hour to 4 hours.

Triethylamine is preferably added after having produced compound (2).

In the manufacturing method of the present invention, compound (2) is synthesized in the form of a monohydrate. When water serving as raw material for the hydrate is added to the reaction solution, a reverse reaction of Step 1 can occur due to the presence of the acid added in Step 1. When this reverse reaction occurs, the residual amount of the starting material in the form of compound (1) present in compound (2) increases. By adding triethylamine after having

produced compound (2), the reverse reaction caused by the acid can be interrupted or the rate at which the reverse reaction proceeds can be decreased. As a result, compound (2) can be crystallized in a state in which the stability of the solution of compound (2) has improved, thereby improving the content of compound (2) in the resulting crystals.

The amount of triethylamine added to the reaction solution is 0.01 equivalents to 1.00 equivalent, and preferably 0.01 equivalents to 0.20 equivalents, with respect to compound (1).

The temperature at which ethyl acetate is added dropwise is 0°C to 60°C and preferably 10°C to 40°C.

The duration of the dropwise addition is 10 minutes to 10 hours and preferably 30 minutes to 4 hours.

(Step 2)

Step 2 is a step for producing compound (3) by reacting compound (2) with acetic anhydride in the presence of an acid.

The acid is preferably added gradually at around room temperature in the presence of compound (2) and acetic anhydride until compound (2) dissolves. This is so that the reaction rate can be controlled.

There are no limitations on the acid used provided it allows acetylation of the hydroxyl groups at the 1-, 2- and 3-positions of the side chain, formation of a carbon-carbon double bond at the 2- and 3-positions of the tetrahydropyran ring, and formation of an oxazoline ring at the 4- and 5-positions of the tetrahydropyran ring to proceed. The acid can be an organic acid such as acetic acid, propionic acid, trifluoroacetic acid and pentafluoropropionic acid, an organic sulfonic acid such as p-toluenesulfonic acid, camphorsulfonic acid and trifluoromethanesulfonic acid, or an inorganic acid such as hydrogen chloride, hydrogen bromide, hydrogen iodide, phosphoric acid, sulfuric acid and nitric acid, is preferably an inorganic acid, and is most preferably sulfuric acid.

The solvent used is preferably a hydrocarbon, and preferably toluene.

The reaction temperature is -20°C to 100°C and preferably -20°C to 60°C.

The reaction time is preferably 30 minutes to 60 hours and more preferably 1 hour to 20 hours.

Triethylamine and then aqueous ammonia are preferably added to the reaction solution containing compound (3) as post-treatment following production of compound (3) to neutralize the reaction solution. In a reaction in which the reaction solution is neutralized with aqueous ammonia, the pH of the resulting reaction solution is preferably 6 to 10 and more preferably 7 to 10.

(Step 3)

Step 3 is a step for producing compound (4) by reacting compound (3) with sodium methoxide.

The solvent used is preferably methanol.

The reaction temperature is preferably -20°C to 70°C and more preferably 0°C to 50°C.

The reaction time is preferably 1 minute to 5 hours and more preferably 5 minutes to 1 hour.

(Step 4)

Step 4 is a step for producing compound (5) by reacting compound (4) with dimethyl carbonate. Compound (5) is produced in the form of crystals.

In Step 4, a base can also be used preferably. There are no limitations on the base provided it can be used in a transformation reaction of a 1,2-diol to a cyclic carbonate. It is preferably an alkali metal alkoxide and more preferably sodium methoxide.

The solvent used is preferably methanol.

The reaction temperature is preferably -30°C to 80°C and more preferably 0°C to 50°C.

The reaction time is preferably 30 minutes to 60 hours and more preferably 1 hour to 20 hours.

In case the purity of the resulting crystals of compound (5) is not sufficiently high, the purity can be increased by purifying with reslurrying in methanol. More specifically, a highly pure compound (5) can be obtained by adding crystals of compound (5) to methanol, heating to 20°C to 60°C and stirring for 1 hour, followed by cooling to room temperature, stirring, filtering the precipitated crystals and washing the crystals with methanol.

(Step 5)

Step 5 is a step for producing compound (6) by reacting compound (5) with dimethyl sulfate in the presence of a base.

The formation of by-products can be inhibited by controlling the reaction rate between compound (5) and dimethyl sulfate. Namely, since the reaction rate can be efficiently controlled by gradually adding the base to the compound (5) and dimethyl sulfate, the formation of by-products can be inhibited, thereby making this preferable. As a result, the

purity of compound (7) obtained by proceeding through the next step, Step 6, can be increased.

There are no limitations on the base used provided it can be used to alkylate hydroxyl groups, and for example, it can be a base indicated in Step 4, is preferably an alkali metal hydride, and is most preferably sodium hydride.

The solvent used is preferably an ether, an amide or a mixture thereof, more preferably tetrahydrofuran, N,N-dimethylacetamide or mixture thereof, and most preferably a mixture of tetrahydrofuran and N,N-dimethylacetamide.

When adding the base to the reaction solution, the reaction temperature is preferably -20°C to 20°C and more preferably -15°C to 15°C.

As the method used to purify compound (6) following production of compound (6), it is preferable to add a solvent immiscible with water to the reaction solution containing compound (6), wash the mixture with an aqueous sodium hydrogencarbonate solution to separate into an organic layer and aqueous layer, and again wash the resulting organic layer with an aqueous sodium hydrogencarbonate solution.

The solvent used is preferably toluene.

(Step 6)

Step 6 is a step for producing compound (7) by reacting compound (6) with azidotrimethylsilane in the presence of titanium (IV) isopropoxide.

The reaction can be carried out at a lower temperature, and therefore more safely, by reacting compound (6) and azidotrimethylsilane in the presence of titanium (IV) isopropoxide, and the desired compound in the form of compound (7) can be synthesized highly selectively from among stereoisomers formed due to orientation differences in the azide group at the 4-position of the tetrahydropyran ring.

The solvent used is preferably an aromatic hydrocarbon, an alcohol or a mixture thereof, more preferably 2-propanol, 2-methyl-2-propanol, toluene or a mixture thereof, and most preferably a mixture of 2-methyl-2-propanol and toluene.

The reaction temperature is preferably -20°C to 80°C and more preferably 0°C to 30°C.

The reaction time is preferably 1 hour to 100 hours and more preferably 5 hours to 30 hours.

After having produced compound (7) by reacting compound (6) and azidotrimethylsilane in the presence of titanium (IV) isopropoxide, as reaction post-treatment, it is preferable to add a hydroxycarboxylic acid to the reaction solution and after that add sodium nitrite in the form of an aqueous solution to the reaction solution.

Although titanium (IV) isopropoxide is a liquid at normal temperatures, when compound (7) is produced by reacting compound (6) with azidotrimethylsilane in the presence thereof, and sodium nitrite is added in the form of an aqueous solution to decompose the residual azidotrimethylsilane, insoluble matter which is hardly soluble, derived from the titanium (IV) isopropoxide is formed. However, the formation of insoluble matter which is hardly soluble, derived from the titanium (IV) isopropoxide can be avoided if a hydroxycarboxylic acid is added to the reaction solution. Consequently, compound (7) can be separated from the titanium (IV) isopropoxide and compounds derived from titanium (IV) isopropoxide simply by filtering, thereby making this preferable. Since the formation of insoluble matter which is hardly soluble, derived from the titanium (IV) isopropoxide can be avoided, a high content of compound (7) can be produced.

The hydroxycarboxylic acid is, for example, lactic acid, tartaric acid or citric acid, preferably lactic acid or tartaric acid, and more preferably lactic acid.

The hydroxycarboxylic acid can be used in the L form, D form or DL form.

The reaction temperature in the reaction in which a hydroxycarboxylic acid is added to the reaction solution is-20°C to 80°C and preferably 0°C to 30°C.

The reaction time is 10 minutes to 100 hours and preferably 30 minutes to 10 hours.

The solvent used to wash the resulting crystals of compound (7) is preferably methanol. The use of methanol results in lower likelihood of the crystals becoming colored.

(Step 7)

Step 7 includes a step for treating compound (7) with triphenylphosphine (Step 7a) and a step for treating the compound obtained in Step 7a with a base and water (Step 7b).

(Step 7a)

The solvent used is preferably tetrahydrofuran or ethyl acetate and more preferably tetrahydrofuran. The procedure is facilitated by adding compound (7) after having dissolved the triphenylphosphine with a solvent, thereby making this preferable.

The reaction temperature is preferably -30°C to 100°C and more preferably 10°C to 60°C.

The reaction time is preferably 30 minutes to 100 hours and more preferably 1 hour to 10 hours.

(Step 7b)

There are no limitations on the base used provided it allows an ester group hydrolysis reaction and cyclic carbonate group elimination reaction to proceed, and the base is preferably an alkali metal hydroxide, more preferably sodium hydroxide or potassium hydroxide, and particularly preferably sodium hydroxide.
The solvent used is preferably tetrahydrofuran, methanol or ethanol, and more preferably tetrahydrofuran.
The acid used to adjust the pH of the reaction mixture to the acidic side is preferably hydrochloric acid.
The reaction temperature is preferably -30°C to 100°C and more preferably 0°C to 70°C.
The reaction time is preferably 10 minutes to 20 hours and more preferably 30 minutes to 10 hours.

(Step 8)

Step 8 is a step for producing compound (10) by reacting compound (8) with N,N'-bis(tert-butoxycarbonyl)-1H-pyrazole-1-carboximidamide (Compound (9)).
Compound (9) can be produced by the method described in Patent Document 3, etc.
In this step, carbonic acid formed as a by-product in Step 7 carried out prior to Step 8 is preferably released in the form of carbon dioxide gas by first adding an acid to the aqueous solution containing compound (8) obtained after synthesizing compound (8) to adjust the pH of the aqueous solution to the acidic side as previously described.
The pH of the reaction solution following addition of acid is preferably 1 to 5.
After removing carbon dioxide gas by addition of acid, the pH of the reaction solution is preferably returned to the alkaline side by adding a base. The pH of the reaction solution following addition of base is preferably 7.5 to 12.0 and more preferably 8.5 to 11.0.
The acid used to adjust the pH of the reaction mixture to the acidic side is preferably hydrochloric acid.
The base used to return the pH of the reaction mixture to the alkaline side is preferably sodium hydroxide.
The solvent used is preferably a mixture of water and an alcohol and more preferably a mixture of water and methanol.
The reaction temperature is preferably -30°C to 80°C and more preferably 0°C to 50°C.
The reaction time is preferably 1 hour to 160 hours and more preferably 5 hours to 80 hours.

(Step 9)

Step 9 is a step for producing compound (11) by heating compound (10) in water.
The solvent used is preferably water.
The reaction temperature is preferably 30°C to 100°C and more preferably 50°C to 100°C.
The reaction time is preferably 30 minutes to 20 hours and more preferably 1 hour to 10 hours.
Slurry purification with water alone is preferable for increasing the purity of the resulting compound (11).
Since compound (11) can be produced in the form of crude crystals not having high purity, crystals of compound (11) of high purity are obtained by adding water to the crude crystals, heating and stirring, followed by cooling, filtering out the resulting crystals, washing and drying.
The temperature during the stirring procedure is preferably 30°C to 100°C and more preferably 50°C to 100°C.
The duration of stirring is preferably 1 hour to 20 hours and more preferably 2 hours to 10 hours.
The purity of the resulting compound (11) can be enhanced by recrystallization by adjusting the pH of the reaction solution containing compound (11). Compound (11) can be crystallized by adding an acid such as hydrochloric acid after putting compound (11) into the state of a slurry with methanol containing compound (11) and water and dissolving it, followed by neutralizing with a base such as sodium hydroxide.
Slurry purification with water alone is more preferable as the method used to purify compound (11).

(Step 10)

Step 10 is a step for producing compound (12) by reacting compound (11) with a $R^1C(OMe)_3$ [wherein $R^1$ represents a $C_1$-$C_{19}$ alkyl group] in the presence of an acid.
The compound represented by the formula $R^1C(OMe)_3$ is preferably 1,1,1-trimethoxyoctane.
There are no limitations on the acid used provided it allows cyclic orthoesterification reaction of a hydroxyl group using an orthoester to proceed. The acid is preferably an organic sulfonic acid or inorganic acid, more preferably p-toluenesulfonic acid, sulfuric acid or hydrogen chloride, and particularly preferably hydrogen chloride.
The solvent used is preferably methanol.
The reaction temperature is preferably -30°C to 80°C and more preferably 0°C to 50°C.
The reaction time is preferably 5 minutes to 20 hours and more preferably 10 minutes to 5 hours.

(Step 11)

Step 11 is a step for producing compound (I) by reacting compound (12) with water in the presence of an acid.

After adding water to the reaction solution containing compound (12) obtained in Step 10 and forming compound (I) by hydrolysis, a base is added to adjust the pH of the reaction solution to 5 to 10 and preferably 6 to 10. Although compound (II) can also be formed together with the forming of compound (I), by adjusting the pH of the reaction solution as described above, compound (I) can be produced with higher selectivity and at a higher yield than compound (II).

The acid used is preferably hydrochloric acid.

The base used to adjust the pH of the reaction solution to the basic side is preferably sodium carbonate.

The reaction temperature is preferably -30°C to 80°C and more preferably 0°C to 50°C.

The reaction time is preferably 1 minute to 100 hours and more preferably 10 minutes to 5 hours.

The 50% by weight particle diameter of compound (I) produced according to the manufacturing method of the present invention, and a pharmacologically acceptable salt thereof, as determined by laser diffraction/scattering particle size distribution measurement is 5 $\mu$M to 15 $\mu$M, while the 90% by weight particle diameter is 15 $\mu$M to 35 $\mu$M.

Here, laser diffraction/scattering particle size distribution measurement (Particle size analysis. Laser diffraction methods) refers to a method for determining particle size distribution by irradiating a group of particles with laser light and calculating the particle size distribution thereof from the intensity distribution pattern of the diffracted/scattered light emitted therefrom. The measurement method is defined in ISO13320 published by the International Organization for Standardization and is standardized internationally. The particle diameters at 50% and 90% of a weight-based cumulative particle size distribution curve obtained by laser diffraction/scattering particle size distribution measurement are respectively defined as the 50% by weight particle diameter and 90% by weight particle diameter.

The neuraminic acid derivative (I) according to the present invention is known to have excellent neuraminidase inhibitory activity and is therefore useful as a drug for treatment or prevention of influenza (refer to the aforementioned Patent Document 1 or 2).

In the case where the neuraminic acid derivative (I) according to the present invention is used as a medicament, especially as a drug for treatment or prevention of influenza, it can be administered orally or parenterally as such, or after mixing with suitable excipients, diluents and the like that are pharmacologically acceptable, and it is preferable that compound (I), which is an active ingredient, is administered in such a manner that it can be directly delivered to the lungs or respiratory tract (including intraoral and intranasal portions).

These pharmaceutical drugs can be produced by well-known methods using additives such as excipients or diluents.

Although the dosage amount varies depending on symptoms, weight, age and the like of the subject to be administered (a warm-blooded animal, preferably a human), it is preferable to administer the neuraminic acid derivative (I) as the active ingredient at 5 mg to 120 mg, preferably 20 mg to 80 mg, and specifically, 20 mg, 40 mg or 80 mg per administration in terms of an anhydride depending on weight and age.

EXAMPLES

The present invention will be described in more detail with reference to the following Examples, Preparation examples and Test examples.

(Example 1)

Step 1: Methyl N-acetylneuraminate monohydrate

Methanol (450 mL), trimethyl orthoformate (138.3 g) and concentrated sulfuric acid (3.4 g) were added to N-acetylneuraminic acid dihydrate (150 g) at room temperature followed by stirring for 2 hours at 30°C. After cooling the reaction solution to 20°C, triethylamine (1.8 g) and water (53 mL) were added followed by adding ethyl acetate (2100 mL) dropwise over 1 hour and stirring for 1 hour. The suspension was further cooled to 5°C and stirred for 1 hour at the same temperature followed by filtering the crystals. The crystals were washed with cold ethyl acetate (300 mL) followed by drying under reduced pressure to give the title compound as a white solid (143.3 g, yield: 96.7%).

MS (FAB): m/z 324 $[M+H]^+$

HRMS (ESI): Exact mass calcd for $C_{12}H_{22}NO_9$ $[M+H]^+$ 324.1295, Found 324.1287

IR (KBr): 3494, 3456, 3267, 2904, 2863, 1753, 1621, 1584, 1299, 1158, 1029, 788, 777, 478 $cm^{-1}$

$^1H$ NMR ($D_2O$, 500 MHz): 1.70 (1H, dd, J = 11.6, 13.0 Hz), 1.84 (3H, s), 2.10 (1H, dd, J = 5.1, 13.0 Hz), 3.34 (1H, dd, J = 1.1, 9.3 Hz), 3.41 (1H, dd, J = 6.2, 11.9 Hz), 3.52 (1H, ddd, J = 2.8, 6.2, 9.3 Hz), 3.62 (1H, dd, J = 2.5, 11.9 Hz), 3.63 (3H, s), 3.71 (1H, dd, J= 10.2, 10.5 Hz), 3.85 (1H, ddd, J = 5.1, 10.2, 11.6 Hz), 3.86 (1H, dd, J = 1.1, 10.5 Hz).

$^{13}C$ NMR ($D_2O$, 125 MHz): 22.1, 38.7, 52.1, 53.6, 63.2, 66.7, 68.3, 70.2, 70.4, 95.4, 171.5, 174.9

(Example 2)

Step 5: Methyl (3aR,4R,7aR)-4-{(S)-methoxy[(4R)-2-oxo-1,3-dioxolan-4-yl]methyl}-2-methyl-3a,7a-dihydro-4H-pyrano[3,4-d][1,3]-oxazole-6-carboxylate

Tetrahydrofuran (240 mL) and N,N-dimethylacetamide (60 mL) were added to the compound obtained in accordance with Step A-4 of Example 1 described in Patent Document 4 (60 g) and suspended followed by cooling to 5°C or lower. After adding dimethyl sulfate (31.8 g) to the suspension, 60% sodium hydride (10.2 g) was added gradually followed by stirring for 3 hours at 3°C. Acetic acid (11.5 g) and toluene (540 mL) were then added to the reaction solution followed by washing the mixture with approximately 7% aqueous sodium hydrogencarbonate solution (240 mL) to separate into an organic layer 1 and an aqueous layer 1. The organic layer 1 was washed with approximately 2% aqueous sodium hydrogencarbonate solution (240 mL) to separate into an organic layer 2 and an aqueous layer 2. The aqueous layer 1 was extracted with toluene (180 mL) to separate an organic layer 3, the aqueous layer 2 was extracted with the organic layer 3 to separate an organic layer 4 that was combined with the organic layer 2. The solvent was then distilled off under reduced pressure until the liquid volume of the combined organic layer became 180 mL to give a toluene solution of the title compound. Step 6: Methyl (2R,3R,4S)-3-acetamide-4-azide-2-{(S)-methoxy[(4R)-2-oxo-1,3-dioxolan-4-yl]methyl}-3,4-dihydro-2H-pyran-6-carboxylate

After adding 2-methyl-2-propanol (60 mL) to the toluene solution of the compound obtained in Step 5, titanium (IV) isopropoxide (16.3 g) and azidotrimethylsilane (37.5 g) were added followed by stirring the mixture for 15 hours at 18°C. Subsequently, lactic acid (36.2 g) was added and stirred for 1 hour at 20°C followed by adding water (120 mL) and approximately 19% aqueous sodium nitrite solution (147.8 g) and stirring for 30 minutes at 25°C. The suspension was then cooled to 5°C or lower followed by stirring for 1 hour at the same temperature and filtering the crystals. After washing the crystals with cold methanol (240 mL), the crystals were dried under reduced pressure to give the title compound as a pale yellow-white solid (62.3 g, yield: 87.9%, stereoisomer ratio: 237).
MS (FAB): m/z 371 $[M+H]^+$
HRMS (ESI): Exact mass calcd for $C_{14}H_{19}N_4O_8$ $[M+H]^+$ 371.12029, Found 371.12018
IR (KBr): 3314, 2106, 1795, 1731, 1668, 1550, 1379, 1285, 1180, 1075 $cm^{-1}$
$^1H$ NMR (DMSO-d6, 500 MHz): 1.89 (3H, s), 3.36 (3H, s), 3.71 (3H, s), 3.88 (1H, dd, J = 1.3, 2.0 Hz), 3.99 (1H, ddd, J = 8.9, 9.2, 10.6 Hz), 4.20 (1H, dd, J = 1.3, 10.6 Hz), 4.29 (1H, dd, J = 2.5, 9.2 Hz), 4.54 (1H, dd, J = 7.9, 12.2 Hz), 4.56 (1H, dd, J= 7.9, 12.2 Hz), 5.06 (1H, ddd, J = 2.0, 7.9, 7.9 Hz), 5.81 (1H, d, J = 2.5 Hz), 8.16 (1H, d, J = 8.9 Hz).
$^{13}C$ NMR (DMSO-d6, 125 MHz): 23.4, 47.0, 53.0, 59.0, 61.7, 66.1, 76.7, 77.7, 79.1, 108.6, 144.7, 155.0, 161.7, 170.1.

The title compound and stereoisomer ratio thereof were measured under the HPLC measurement conditions indicated below.

HPLC Measurement Conditions (1)

Column: Column packed with 3 μm octadecylsilylated silica gel for use in liquid chromatography in a stainless steel tube having an inner diameter of 4.6 mm and length of 25 cm (Cadenza CD-C18, manufactured by Imtakt, 4.6 x 250 mm, 3 μm)
Column temperature: 30°C
Measurement wavelength: 210 nm
Mobile phase A: 0.05 mol/L phosphate buffer solution (pH 3)
Mobile phase B: Acetonitrile

Gradient conditions:

0 to 25 min: mobile phase A: mobile phase B = 75:25
25 to 40 min: mobile phase ratio changed to mobile phase A: mobile phase B = 47.5:52.5
40 to 65 min: mobile phase A: mobile phase B = 47.5:52.5
[Note, however,that, the 0.05 mol/L phosphate buffer solution (pH 3) of mobile phase A indicates a buffer solution obtained by adding 0.05 mol/L phosphoric acid to a 0.05 mol/L aqueous potassium dihydrogenphosphate solution and adjusting the pH to 3.]
Flow rate: 0.7 mL/min
Sample concentration: Approximately 500 μg/L

Injection amount: 5 μL
Retention time of the title compound: Approximately 22 min
Retention time of the stereoisomer: Approximately 23 min

Stereoisomer ratio = Peak area of compound (7)/peak area of stereoisomer

Step 7: (2R,3R,4S)-3-acetamide-4-amino-2-[(1R,2R)-2,3-dihydroxy-1-methoxypropyl]-3,4-dihydro-2H-pyran-6-carboxylic acid

Triphenylphosphine (39.0 g) and tetrahydrofuran (200 mL) were added to the compound obtained in Step 6 (50 g) at room temperature followed by stirring the mixture for 1 hour at 20°C, stirring for 1 hour at 40°C and cooling to 10°C or lower. Approximately 10.2% aqueous sodium hydroxide solution (166.4 g) was added to the reaction solution followed by heating to 40°C and stirring for 2 hours at the same temperature. After cooling the reaction solution to 25°C or lower, concentrated hydrochloric acid (28.6 g) and ethyl acetate (150 mL) were added followed by allowing to stand undisturbed and then separating the aqueous layer to give an aqueous solution of the title compound.

Step 8: (2R,3R,4S)-3-acetamide-4-[2,3-bis(tert-butoxycarbonyl)guanidino]-2-[(1R,2R)-2,3-dihydroxy-1-methoxypropyll-3,4-dihydro-2H-pyran-6-carboxylic acid

After adding concentrated hydrochloric acid to the aqueous solution of the compound obtained in Step 7 to adjust the pH to 2.7 and removing carbon dioxide gas from the system, an aqueous sodium hydroxide solution was added to adjust the pH to 9.5. N,N'-Bis(tert-butoxycarbonyl)-1H-pyrazole-1-carboximidamide (46.1 g) and methanol (300 mL) were added to the aqueous solution followed by heating the suspension to 23°C and stirring for 46 hours at the same temperature. The solvent was distilled off under reduced pressure until the liquid volume of the reaction suspension became approximately 400 mL followed by adding ethyl acetate (165 mL) and distilling off the solvent under reduced pressure to a liquid volume of approximately 400 mL. Ethyl acetate (355 mL) was added to the resulting liquid followed by allowing to stand undisturbed and then separating the aqueous layer, after which ethyl acetate (250 mL) was again added followed by allowing to stand undisturbed and then separating the aqueous layer. Ethyl acetate (350 mL) was added to the resulting aqueous solution, and after adjusting the pH to 2.7 with concentrated hydrochloric acid, the reaction solution was allowed to stand undisturbed to separate into an organic layer 1 and an aqueous layer 1. Ethyl acetate (175 ml) was then added to the aqueous layer 1 followed by allowing to stand undisturbed to separate an organic layer 2. The resulting organic layer 1 and organic layer 2 were combined and the solvent was distilled off under reduced pressure to a liquid volume of approximately 200 mL. Water (150 mL) was then added to the concentrated solution followed by distilling off the solvent under reduced pressure to a liquid volume of approximately 150 mL and adding water (100 mL) to give an aqueous solution of the title compound.

Step 9: (2R,3R,4S)-3-acetamide-2-[(1R,2R)-2,3-dihydroxy-1-methoxypropyl]-4-guanidino-3,4-dihydro-2H-pyran-6-carboxylic acid

The aqueous solution of the compound obtained in Step 8 was stirred for 4 hours at 80°C. After cooling the reaction solution to 30°C or lower and adding methanol (500 mL), the mixture was stirred for 1 hour followed by filtering the crystals. After washing the crystals with methanol (100 mL), the crystals were dried under reduced pressure to give the title compound as a white solid (36.3 g, yield: 77.6%). The crude title compound (30 g) was then suspended by adding water (120 mL) followed by heating to 96°C. After stirring the suspension for 3.5 hours at the same temperature, the suspension was cooled to 30°C or lower followed by adding methanol (90 mL) and stirring for 1 hour. After filtering the suspension and washing the crystals with methanol (60 mL), the crystals were dried under reduced pressure to give the title compound as a white solid (29.1 g, yield: 97.1%).
MS (FAB): m/z 347$[M+H]^+$
Anal. calcd for $C_{13}H_{22}N_4O_7$ : C, 45.08; H, 6.40; N, 16.18. Found C, 44.85; H, 6.16; N, 16.09.
IR (KBr): 3440, 3375, 3256, 1699, 1653, 1587, 1401, 1329, 1284, 1171, 1087, 1029 $cm^{-1}$
$^1H$ NMR ($D_2O$, 500 MHz): 1.94 (3H, s), 3.31 (3H, s), 3.45 (1H, dd, J = 1.5, 8.6 Hz), 3.57 (1H, dd, J = 5.6, 12.0 Hz), 3.78 (1H, dd, J = 3.0, 12.0 Hz), 3.88 (1H, ddd, J = 3.0, 5.6, 8.6 Hz), 4.10 (1H, dd, J= 9.7, 9.7 Hz), 4.30 (1H, dd, J = 1.5, 9.7 Hz), 4.30 (1H, dd, J = 2.2, 9.7 Hz), 5.52 (1H, d, J = 2.2 Hz). $^{13}C$ NMR ($D_2O$, 125 MHz): 22.1, 47.7, 51.8, 60.5, 62.5, 69.6, 75.7, 77.8, 104.0, 149.4, 157.0, 169.0, 174.2.

Step 10: (2R,3R,4S)-3-acetamide-4-guanidino-2-{(S)-[(2RS,4R)-2-heptyl-2-methoxy-1,3-dioxolan-4-yl](methoxy)methyl}-3,4-dihydro-2H-pyran-6-carboxylic acid

Methanol (50 mL), 1,1,1-trimethoxyoctane(trimethyl orthooctanoate) (17.70 g) and 9.2% hydrogen chloride-methanol solution (13.64 g) were added to the compound (10 g) obtained in Step 9 followed by stirring for 1 hour at 25°C. The solvent was distilled off under reduced pressure to a liquid volume of about 35 mL to give a methanol solution of the title compound. Step 11: (2R,3R,4S)-3-acetamide-4-guanidino-2-[(1R,2R)-2-hydroxy-1-methoxy-3-(octanoyloxy)propyl]-3,4-dihydro-2H-pyran-6-carboxylic acid monohydrate [compound (I)] and (2R,3R,4S)-3-acetamide-4-guanidino-2-[(1S,2R)-3-hydroxy-1-methoxy-2-(octanoyloxy)propyl]-3,4-dihydro-2H-pyran-6-carboxylic acid monohydrate [Compound (II)]

Water (100 mL) was added to the methanol solution of the compound obtained in Step 10 followed by washing the reaction solution twice with ethyl acetate (50 mL) to separate the aqueous layer. The pH of the reaction solution was adjusted to pH 7.2 with 17% aqueous sodium carbonate solution, and after stirring the reaction solution for 30 minutes, the pH was adjusted to 8.8 with 17% aqueous sodium carbonate solution followed by stirring for 3 hours. Next, the pH of the reaction solution was adjusted to pH 5.3 with concentrated hydrochloric acid followed by cooling to 5°C or lower, stirring for 1 hour and filtering the crystals. The crystals were then washed with water (50 mL) followed by drying under reduced pressure to give the crude title compound as white crystals (13.59 g, yield: 95.9%). Methanol (60 mL) was then added to the crude title compound (10 g) to dissolve it followed by gradually adding water (120 mL) to the solution at 25°C, cooling to 5°C or lower, stirring for 1 hour and filtering the crystals. After washing the crystals with 33% aqueous methanol solution (30 mL), the crystals were dried under reduced pressure to give the title compound as white crystals (9.62 g, yield: 96.2%, chemical purity: 99.91%, ratio of compound (I):compound (II) = 97:3, particle diameter: 50% by weight particle diameter = 8.8 $\mu$M, 90% by weight particle diameter = 25.4 $\mu$M).
MS (FAB): m/z 473$[M+H]^+$
KF moisture content: 3.9%
Anal. calcd for $C_{21}H_{36}N_4O_8.1.065H_2O$: C, 51.29; H, 7.82; N, 11.39. Found C, 51.21; H, 7.82; N, 11.32.
IR (KBr): 3334, 3289, 2929, 1736, 1665, 1640, 1401, 1325, 1283, 1173, 1114 $cm^{-1}$
$^1H$ NMR ($CD_3OD$, 500 MHz): 0.88 (3H, t, J = 7.0 Hz), 1.25 -1.34 (8H, m), 1.62 (2H, tt, J = 7.2, 7.5 Hz), 1.99 (3H, s), 2.35 (2H, t, J = 7.5 Hz), 3.38 (3H, s), 3.45 (1H, dd, J= 2.5, 8.2 Hz), 4.09 -4.14 (2H, m), 4.23 (1H, dd, J = 9.0, 9.0 Hz), 4.29 - 4.36 (3H, m), 5.55 (1H, d, J = 2.5 Hz).
$^{13}C$ NMR ($CD_3OD$, 125 MHz): 13.1, 21.5, 22.3, 24.7, 28.8, 28.9, 31.5, 33.7, 47.8, 51.4, 60.0, 65.5, 67.4, 76.1, 78.9, 102.3, 150.3, 157.6, 168.1, 172.2, 174.1

(Reference Example 1)

Methyl N-acetylneuraminate monohydrate

Methanol (800 mL), trimethyl orthoformate (37.7 g) and concentrated sulfuric acid (2.5 g) were added to N-acetylneuraminic acid (100 g) at room temperature followed by stirring for 5 hours at 40°C. N,N-Dimethylacetamide (100 mL) was then added to the reaction solution followed by distilling off the solvent under reduced pressure to a liquid volume of 400 mL. After cooling the concentrated solution to 20°C or lower, water (50 mL) was added and ethyl acetate (1800 mL) was added dropwise over 1 hour followed by stirring for 1 hour. After further cooling the suspension to 5°C, the suspension was stirred for 1 hour at the same temperature followed by filtering the crystals. The crystals were washed with cold ethyl acetate (200 mL) followed by drying under reduced pressure to give the title compound as a white solid (104.8 g, yield: 94.9%).

## Claims

1. A method for manufacturing a compound represented by the formula (10):

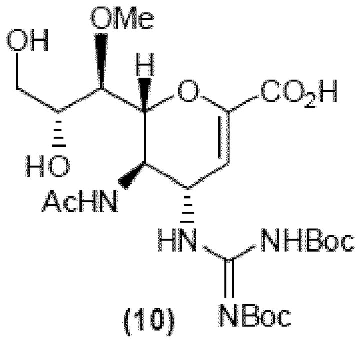

(10)

by reacting a compound represented by the formula (7):

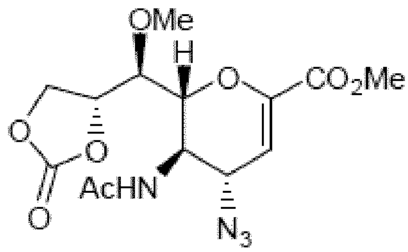

(7)

with triphenylphosphine followed by reacting with a base and water to produce a compound represented by the formula (8):

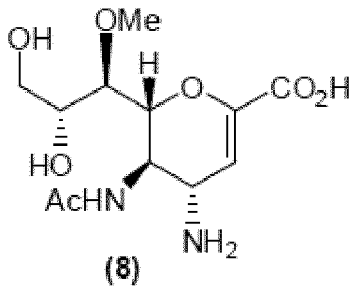

(8)

,

and adding an acid to an aqueous solution containing the compound represented by the formula (8) to remove carbonic acid and reacting the compound represented by the formula (8) with a compound represented by the formula (9):

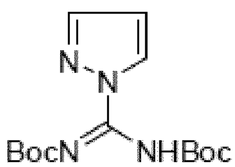

(9)

in the aqueous solution from which carbonic acid has been removed.

**2.** A method for manufacturing a compound represented by the formula (I), or a pharmacologically acceptable salt thereof:

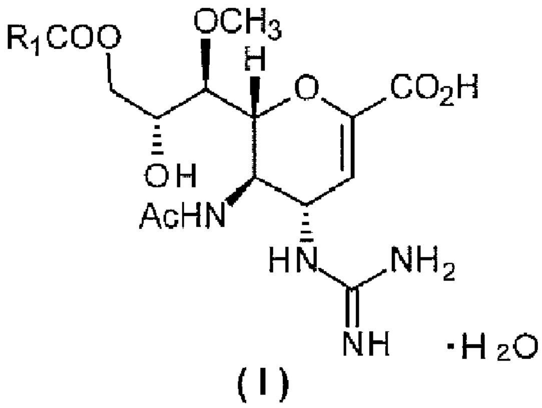

(I)

wherein $R^1$ is a $C_1$-$C_{19}$ alkyl group, **characterized by** comprising the manufacturing method according to claim 1.

**3.** The manufacturing method according to claim 2, wherein $R^1$ is a 1-heptyl group.

Europäisches Patentamt
European Patent Office
Office européen des brevets

# EUROPEAN SEARCH REPORT

Application Number
EP 15 17 4323

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2008/126943 A2 (DAIICHI SANKYO CO LTD [JP]; NAKAMURA YOSHITAKA; MURAKAMI MASAYUKI; YAM) 23 October 2008 (2008-10-23) * the whole document; in paticular, pages 50-52, (Step A-7) and (Step-A-8), and pages 66-67, example 1, step A-7 and step A-8 * ----- | 1-3 | INV. C07D309/28 |

TECHNICAL FIELDS SEARCHED (IPC)
C07D

1

The present search report has been drawn up for all claims

| Place of search | Date of completion of the search | Examiner |
|---|---|---|
| Munich | 8 October 2015 | Fink, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 17 4323

This annex lists the patent family members relating to the patent documents cited in the above-mentioned European search report. The members are as contained in the European Patent Office EDP file on
The European Patent Office is in no way liable for these particulars which are merely given for the purpose of information.

08-10-2015

| Patent document cited in search report | Publication date | Patent family member(s) | Publication date |
|---|---|---|---|
| WO 2008126943 A2 | 23-10-2008 | BR PI0809601 A2 | 14-10-2014 |
| | | CA 2683065 A1 | 23-10-2008 |
| | | CA 2783587 A1 | 23-10-2008 |
| | | CA 2783620 A1 | 23-10-2008 |
| | | CA 2783688 A1 | 23-10-2008 |
| | | CN 101679339 A | 24-03-2010 |
| | | CN 102603691 A | 25-07-2012 |
| | | CN 102603722 A | 25-07-2012 |
| | | CN 102617295 A | 01-08-2012 |
| | | CN 102850311 A | 02-01-2013 |
| | | EP 2132191 A2 | 16-12-2009 |
| | | EP 2360135 A1 | 24-08-2011 |
| | | EP 2360151 A1 | 24-08-2011 |
| | | EP 2360156 A1 | 24-08-2011 |
| | | ES 2396203 T3 | 19-02-2013 |
| | | ES 2404411 T3 | 27-05-2013 |
| | | ES 2413704 T3 | 17-07-2013 |
| | | ES 2445715 T3 | 04-03-2014 |
| | | HK 1138594 A1 | 21-06-2013 |
| | | HK 1159084 A1 | 09-08-2013 |
| | | HK 1159085 A1 | 01-08-2014 |
| | | IL 201264 A | 31-08-2015 |
| | | IL 224943 A | 30-04-2014 |
| | | IL 224944 A | 30-04-2014 |
| | | JP 5249947 B2 | 31-07-2013 |
| | | JP 5686420 B2 | 18-03-2015 |
| | | JP 2010523472 A | 15-07-2010 |
| | | JP 2013144701 A | 25-07-2013 |
| | | JP 2014208698 A | 06-11-2014 |
| | | KR 20100015460 A | 12-02-2010 |
| | | KR 20140022471 A | 24-02-2014 |
| | | KR 20140032481 A | 14-03-2014 |
| | | KR 20150006075 A | 15-01-2015 |
| | | KR 20150046361 A | 29-04-2015 |
| | | TW 200846351 A | 01-12-2008 |
| | | TW 201350492 A | 16-12-2013 |
| | | TW 201350493 A | 16-12-2013 |
| | | TW 201350494 A | 16-12-2013 |
| | | US 2010035947 A1 | 11-02-2010 |
| | | US 2013197072 A1 | 01-08-2013 |
| | | US 2014018417 A1 | 16-01-2014 |
| | | US 2015259314 A1 | 17-09-2015 |
| | | WO 2008126943 A2 | 23-10-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6340702 B
- JP 3209946 B
- US 6844363 B
- JP 3920041 B
- WO 0180892 A
- JP 4205314 B
- US 12450699 B
- WO 2008126943 A
- JP SHO4994768 B

### Non-patent literature cited in the description

- **T. HONDA et al.** *Bioorganic Medicinal Chemistry Letters,* 2002, 1921-1924
- **T. HONDA et al.** *Bioorganic Medicinal Chemistry Letters,* 2002, 1925-1928